# EUROPEAN PATENT SPECIFICATION

(11) **EP 2 778 174 B1**
(45) Date of publication and mention of the grant of the patent: **01.11.2017**
(21) Application number: 12848401.1
(22) Date of filing: 08.11.2012
(51) Int. Cl.: C07K 16/18, G01N 33/53, C12N 15/00, G01N 33/68

(54) **ANTIBODY SPECIFIC TO PROFILAGGRIN C-TERMINAL DOMAIN, AND USE THEREOF**
SPEZIFISCHER ANTIKÖRPER GEGEN PROFILAGGRIN C-TERMINALE DOMÄNE UND SEINE VERWENDUNG
ANTICORPS SPÉCIFIQUE DU DOMAINE C TERMINAL DE LA PROFILAGGRINE ET SON UTILISATION

(30) Priority: 09.11.2011 JP 2011245483
(43) Date of publication of application: 17.09.2014
(73) Proprietor: Shiseido Company, Ltd., Tokyo 104-0061 (JP)
(72) Inventor: HIBINO, Toshihiko, Yokohama-shi Kanagawa 236-8643 (JP); YAMAMOTO, Mami, Tokyo 160-0023 (JP); TAKAGI, Masaya, Yokohama-shi Kanagawa 236-8643 (JP); SAKABE, Junichi, Singapore 423912 (SG); TOKURA, Yoshiki, Hamamatsu-shi Shizuoka 4313192 (JP)
(74) Representative: Marro, Nicolas
(86) International application number: PCT/JP2012/079015
(87) International publication number: WO 2013/069741

(56) References cited:
- WO-A1-2011/068166
- LUDOVIC CHERVET ET AL: "Missing C-terminal filaggrin expression, NFkappaB activation and hyperproliferation identify the dog as a putative model to study epidermal dysfunction in atopic dermatitis", EXPERIMENTAL DERMATOLOGY, vol. 19, no. 8, 8 August 2010 (2010-08-08) , pages e343-e346, XP055067522, ISSN: 0906-6705, DOI: 10.1111/j.1600-0625.2010.01109.x
- FALLON PG. ET AL.: 'A homozygous frameshift mutation in the mouse Flg gene facilitates enhanced percutaneous allergen priming' NAT. GENET. vol. 41, no. 5, 2009, pages 602 - 608, XP055067518
- DATABASE GENBANK [Online] 06 November 2011 IRVINE AD. ET AL.: 'Definition: Homo sapiens filaggrin (FLG)', XP003033827 Database accession no. NM_002016
- JUN'ICHI SAKABE: 'Filaggrin Idenshi Hen'i ni yotte Naze Filaggrin ga Teika suru noka : C Mattan Ryoiki no Nazo' SKIN RESEARCH, 2011 NEN 10 GATSU vol. 10, no. 16, pages 25 - 28, XP008174081
- CHERVET L. ET AL.: 'Missing C-terminal filaggrin expression, NFkappaB activation and hyperproliferation identify the dog as a putative model to study epidermal dysfunction in atopic dermatitis' EXP. DERMATOL. vol. 19, no. 8, 2010, pages E343 - E346, XP055067522
- ROBERT GRUBER ET AL: "Filaggrin mutations p.R501X and c.2282del4 in ichthyosis vulgaris", EUROPEAN JOURNAL OF HUMAN GENETICS, vol. 15, no. 2, 1 February 2007 (2007-02-01), pages 179-184, XP055188840, ISSN: 1018-4813, DOI: 10.1038/sj.ejhg.5201742

## Description

### TECHNICAL FIELD

The present invention provides a method for detecting a mutation in a filaggrin gene using an antibody specific to a C-terminal domain of profilaggrin.

### BACKGROUND ART

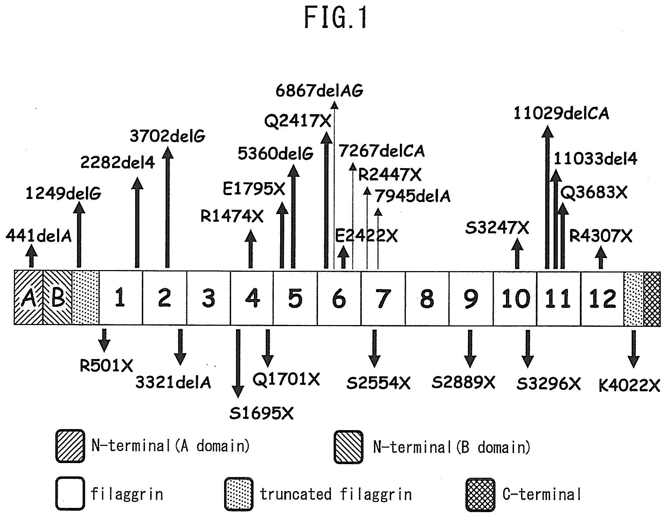

Keratin fibers of the granular layer of the skin bind to a protein referred to as filaggrin and aggregate during keratinization, and form a unique pattern referred to as a "keratin pattern". A precursor substance of filaggrin is referred to as profilaggrin and is stored in keratohyalin granules within granular cells. The profilaggrin has a structure comprising filaggrin repeats consisting of 10 to 12 continuous filaggrin molecules interposed between the N-terminal domain and the C-terminal domain (FIG. 1). Along with keratinization, these domains, etc., are cut out and individual filaggrin molecules are produced. When the filaggrin finally degrades, a natural moisturizing factor (NMF) is formed. The constituent components of NMF are amino acids and their derivatives, which are known to play an important role in maintaining the moisture of the skin (Horii I, Kawasaki K, Koyama J et al. J Dermatol. 1983; 10: 25-33).

Mutations in filaggrin cause a reduction of NMF, and as a result, cause a decrease in the water-retaining capability of the horny layer. However, recent research has indicated that a genetic mutation of filaggrin is the cause of ichthyosis and some atopic dermatitis (Sandilands A, Terron-Kwiatkowski A, Hull PR, et al. Nat Genet 2007; 39: 650 to 654), surprisingly revealing that the mutations in filaggrin not only reduce the water-retaining capability of the horny layer resulting from the decrease in NMF, but also directly influence a barrier function.

The aforementioned diseases result from the abnormalities in the barrier function of the horny layer, as described in the guidelines for the treatment of atopic dermatitis reported in the Journal of the Japan Dermatological Association in 2010 (Norito Kato, 'Treatment Guidelines and New Treatments for Atopic Dermatitis", Journal of the Japanese Dermatological Association 2010, 120(13), 2564-2565), which states that "the pathological conditions of atopic dermatitis accompany a physiological abnormality of the skin, such as dry skin and barrier function abnormality resulting from the abnormalities in the epidermis, specifically the horny layer".

Subsequent research has estimated that approximately half of atopic dermatitis in Europe and approximately 30% of atopic dermatitis in Japan result from genetic mutations of filaggrin. From this point, it is sufficiently understood that atopic dermatitis is a multifactorial disease. However, it is critically important to judge the presence or absence of a mutation in the filaggrin gene for decision on courses of future treatment. Further, regarding a test subject free of atopic dermatitis, it will become possible to predict that the subject is highly likely to suffer from atopic dermatitis in the future if a mutation in a filaggrin gene is detected.

However, numerous mutations in the filaggrin gene have been reported. Specifically, such mutations do not exist in a specific region, but exist almost over the entirety (FIG. 1). Further, it has been reported that filaggrin repeats are unlikely to be produced if there is a mutation in the filaggrin gene itself or in a part of the C-terminal region of a gene encoding profilaggrin, which is a precursor of filaggrin (Sandilands A, Terron-Kwiatkowski A, Hull PR et al. Nat Genet 2007; 39: 650-654). Therefore, in order to specify whether or not there is a mutation in filaggrin, it is necessary to sequence the entire profilaggrin gene. However, even current genetic analysis technology cannot easily analyze the profilaggrin gene. Ludovic, C. et al. 2010 Exp. Derm. 19:e343-e346 teaches the use of commercial antibodies directed against the C-terminal domain of dog profilaggrin in the immunohistochemical diagnosis of atopic dermatitis in skin.

For example, filaggrin repeats, which are the most important region in the profilaggrin gene, are slightly different from each other, and all the filaggrin repeats must be sequenced accurately. Further, the mutations in a filaggrin gene vary depending on an ethnic group. As a result, it is remarkably difficult to determine that a difference of one base is a mutation, due to the variation in the various individual repeats in these various regions, and moreover, due to the ethnic variation. Therefore, currently, mutations in a profilaggrin gene can be analyzed in only specific facilities in the world.

### PRIOR ART DOCUMENTS

### NON-PATENT LITERATURE

Non-patent literature 1: Horii I, Kawasaki K, Koyama J, etal, J Dermatol. 1983: 10: 25-33
Non-patent literature 2: Sandilands A, Terron-Kwiatkowski A, Hull PR, et al. Nat Genet 2007; 39: 650-654
Non-patent literature 3: Kato, Norito "Treatment Guidelines and New Treatments for Atopic Dermatitis", Journal of the Japanese Dermatological Association 2010, 120(13), 2564-2565

### SUMMARY OF THE INVENTION

### PROBLEMS TO BE SOLVED BY THE INVENTION

The object of the present invention is to provide a new method for detecting a mutation in a filaggrin gene using an antibody specific to a C-terminal domain of profilaggrin.

### MEANS FOR SOLVING THE PROBLEMS

The present inventors have discovered that a filaggrin gene mutation can be detected non-invasively and simply, using an antibody specific to the C-terminal domain, and have completed the present invention.

Therefore, the present application includes the following invention: A method for detecting a mutation in a filaggrin gene, comprising the steps of:
detecting the presence or absence of a C-terminal domain of a human profilaggrin protein in a skin sample of a subject with an antibody specific to said C-terminal domain, wherein the C-terminal domain is a peptide comprising the amino acid sequence set forth in SEQ ID NO:1, and
judging that the mutation in a filaggrin gene is present in the subject, in case when said C-terminal domain is not detected,
wherein said skin sample is a horny layer sample obtained by tape stripping.

### EFFECT OF THE INVENTION

If a mutation is generated in a profilaggrin gene, filaggrin repeats following the mutation, and eventually, the C-terminal domain of the profilaggrin cannot be created. According to the present invention, the presence or absence of the C-terminal domain within the skin sample can be detected using an antibody specific to the domain to simply detect the presence or absence of a filaggrin gene mutation. Further, a profilaggrin gene mutation may be a cause of atopic dermatitis, and thus, the diagnosis or prediction of atopic dermatitis using the antibody of the present invention can be performed non-invasively and simply.

### BRIEF DESCRIPTION OF THE DRAWINGS

[FIG. 1] is a schematic illustration showing a structure of the profilaggrin gene (Chrlq21.3/Human) having 12 filaggrin repeats, and mutation sites of the gene.

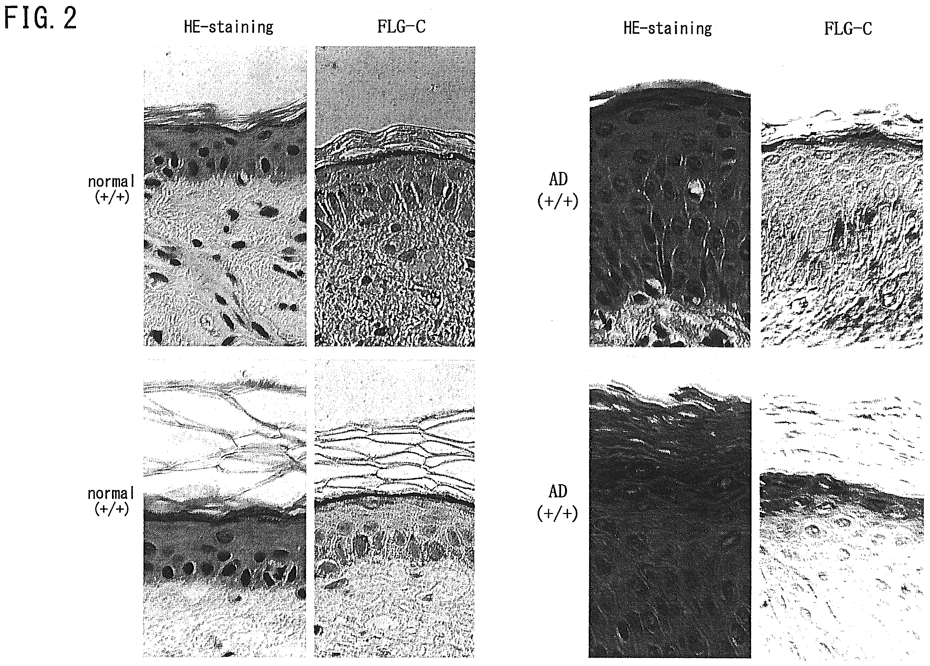

[FIG. 2] is an immunostaining diagram of normal skin (normal (+/+)) without any profilaggrin gene mutations and of atopic dermatitis skin (AD(+/+)). The drawings on the left side show the results of staining with Hematoxylin-Eosin (HE staining), and the drawings on the right side show the results of staining with an antibody specific to the C-terminal domain of profilaggrin and a chromogenic substrate DAB (3,3'-diaminobenzidine) (FLG-C).

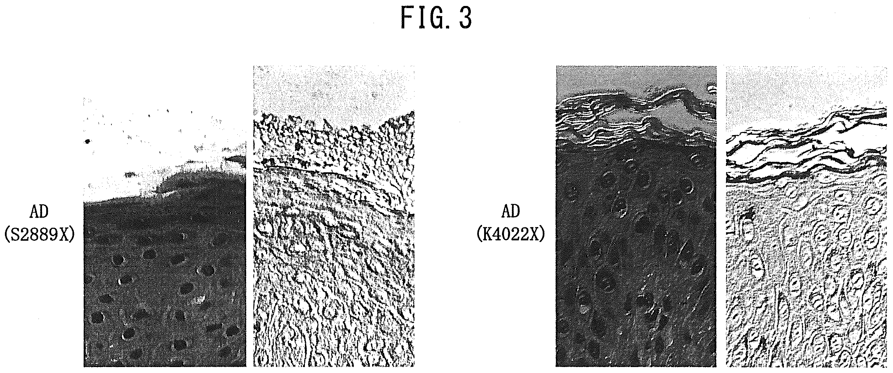

[FIG. 3] is an immunostaining diagram of atopic dermatitis skin with profilaggrin gene mutations (S2889X; K4022X). The drawings on the left side show the results of staining with Hematoxylin-Eosin (HE staning), and the drawings on the right side show the results of staining with the antibody specific to the C-terminal domain of profilaggrin and a chromogenic substrate DAB (3,3'-diaminobenzidine) (FLG-C).

### EMBODIMENT FOR CARRYING OUT THE INVENTION

According to one aspect of the present description, there is provided an antibody specific to a C-terminal domain of a human profilaggrin gene, wherein the C-terminal domain is a peptide comprising the amino acid sequence set forth in SEQ ID NO:1 or a variant thereof. Filaggrin is roughly categorized into Type I and Type II. However, the term "profilaggrin" as used herein refers to Type I. Profilaggrin has a structure comprising filaggrin repeats consisting of 10 to 12 continuous filaggrin molecules interposed between the N-terminal domain and the C-terminal domain (FIG. 1). SEQ ID NO:2 indicates the entire sequence of a C-terminal domain consisting of 157 amino acids.

With respect to the C-terminal domain, the amino acid sequence set forth in SEQ ID NO:1 is not homologous to either Type II profilaggrin or hornerin, which is a profilaggrin-analogous molecule, and is a sequence specific to Type I. Therefore, an antibody specific to the amino acid sequence does not cross-react with these analogous proteins.

The variant may be any variant as long as an antibody obtained using the variant as an antigen does not recognize a peptide having a sequence analogous to the amino acid sequence set forth in SEQ ID NO:1 in the skin and is specific to the C-terminal domain of a human profilaggrin gene. For example, the variant may be a peptide comprising an amino acid sequence derived from the amino acid sequence set forth in SEQ NO:1 by deletion, substitution, or addition of one or more amino acids.

While not limited thereto, the peptide can be obtained by chemically synthesis according to a conventional method. The antibody of the present description can be obtained by administering the peptide as the antigen, preferably an antigen complex comprising the peptide bound to a suitable carrier, to mammals, for example, rats, mice, rabbits, etc. While not limited thereto, the dosage amount of the antigen or antigen complex per animal is 0.1 to 100 mg when no adjuvant is used, and is 10 to 1000 µg when an adjuvant is used. Examples of the adjuvant include Freund's Complete Adjuvant (FCA), Freund's Incomplete Adjuvant (FIA), aluminum hydroxide adjuvant, etc.

The immunization is mainly performed by intravenous, subcutaneous, or intraperitoneal injection. Further, the immunization interval is not specifically limited. However, immunization is performed 1 to 10 times, preferably 2 to 5 times, at an interval of several days to several weeks, preferably, an interval of 2 to 5 weeks. Moreover, the antibody titer is measured by Western blotting, Enzyme-Linked Immunosorbent Assay (ELISA or EIA), Radioimmunoassay (RIA), etc., 6 to 60 days after the day of the final immunization. Preferably, blood is collected and antiserum is obtained on the day when the maximum antibody titer is exhibited.

The polyclonal antibody may be purified by affinity chromatography using a column to which the antigen peptides are bound, and other purification methods known to a person skilled in the art, for example, ion exchange chromatography, gel electrophoresis chromatography, high-performance liquid chromatography, etc. Further, the antibody of the present description may be a monoclonal antibody. The monoclonal antibody may be prepared according to an already known method using a peptide having the amino acid sequence set forth in SEQ ID NO:1 or a variant thereof.

The present invention provides a method for detecting a mutation in a filaggrin gene, wherein the method comprises the steps of: detecting the presence or absence of a C-terminal domain of a human profilaggrin protein in a skin sample of a subject with an antibody specific to said C-terminal domain, wherein the C-terminal domain is a peptide comprising the amino acid sequence set forth in SEQ ID NO:1, and judging that the mutation in a filaggrin gene is present in the subject, in case when said C-terminal domain is not detected, wherein said skin sample is a horny layer sample obtained by tape stripping. The term "a mutation in a filaggrin gene" as used herein refers to one or more mutations of a filaggrin gene, which inhibit the formation of the C-terminal domain of profilaggrin. Examples of the mutations include the mutation S2889X in which termination occurs at the serine in position 2889, the mutation K4022X in which termination occurs at the lysine in position 4022, etc. (FIG. 1).

A mutation in the profilaggrin gene leads to atopic dermatitis. Therefore, according to the method of the present invention, when the C-terminal domain is not detected, it is judged that the subject is suffering or is highly likely to suffer from atopic dermatitis.

The detection of the presence or absence of the C-terminal domain is carried out by an immunological method well known to a person skilled in the art, for example, immunoassay (ELISA, RIA, etc.) The skin samples are collected by any method from the arms, legs, head, etc., of subjects. The subjects are mammals, preferably humans with suspected skin diseases caused by filaggrin gene mutations, specifically, atopic dermatitis. A tape stripping method is simple and non-invasive. The tape stripping is a method for collecting a horny layer sample, which comprises bonding a piece of adhesive tape to the skin surface layer and peeling off the piece so that the skin horny layer adheres to the peeled adhesive tape. According to the tape stripping method, a skin sample containing a profilaggrin gene can be simply obtained, and a filaggrin gene mutation can be detected non-invasively.

A preferable method of tape stripping is as follows: First, a surface layer of skin is cleaned with, for example, ethanol, etc., to remove sebum, dirt, and the like. A piece of adhesive tape cut to a suitable size (for example 5 x 5 cm) is gently placed on the skin surface. The entire piece of tape is pressed flat onto the skin surface by applying uniform pressure, and the adhesive tape is subsequently peeled off while applying uniform force. The adhesive tape may be a commercially available cellophane tape, for example, Scotch Superstrength Mailing Tape (manufactured by 3M), Cellophane Tape (CelloTape(R), Nichiban), etc.

A neutral to weakly alkaline buffer including a non-ionic surfactant can be used to extract a soluble component from the tape-stripped horny layer, and includes, but not limited to the following buffer: 100 mM Tris HCl + 0.14 M NaCl + 0.1% Triton ×100]. The tape having a horny layer adhering thereto is cut into a strip with scissors, etc., transferred to a container, such as an Eppendorf tube, immersed in a small amount of the aforementioned buffer, stirred by inversion, to efficiently extract the soluble components. The extracted soluble components are subjected to an immunoassay, etc., using the antibody of the present description, to judge the presence or absence of a profilaggrin gene mutation.

According to one aspect of the present description, there is provided a kit for detecting a mutation in a filaggrin gene, comprising the antibody. The kit may comprise the antibody of the present description in a container. The kit may further comprise reagents required for the aforementioned detection method, for example, in case of the detection of a mutation by the ELISA method, an enzyme conjugate, substrate solution, quenching solution, wash solution, enzyme conjugate diluent, assay buffer, control, sample diluent, conjugate, etc., and operating instructions explaining the procedures of the method.

The present invention will be specifically explained below in more detail with reference to specific examples. However, the present invention is not limited thereto.

### EXAMPLES

### Immunohistochemical Staining

Skin section samples were prepared from normal skin and atopic dermatitis skin, which were free of a profilaggrin gene mutation, and atopic dermatitis skin with a profilaggrin gene mutation (S2889X or K422X). The immunohistochemical staining of the skin fragment was performed by the method described in Kamata et al. (J. Biol. Chem., Vol. 284, Issue 19, 12829-12836, May 8, 2009). The antibody specific to the profilaggrin C-terminal domain was obtained by immunizing a rabbit with a synthetic peptide having the amino acid sequence CKASAFGKDHPRYYATYINKDP as the immunogen (manufactured by Sigma Inc.)

The normal skin and atopic dermatitis skin, which were free of a profilaggrin gene mutation, were stained with Hematoxylin-Eosin, or with the antibody specific to a profilaggrin C-terminal domain and a chromogenic substrate DAB (3,3'-diaminobenzidine). The results are shown in FIG. 2. Profilaggrin was localized in the granular layer. The color development of DAB staining in the granular layer was reduced in the atopic dermatitis skin free of a profilaggrin gene mutation compared to normal skin (refer to the immunostaining diagram of FLG-C). Namely, the results of FIG. 2 reveal that the expression of filaggrin is reduced in atopic dermatitis skin even free of a profilaggrin gene mutation.

Next, immunostaining was performed for atopic dermatitis skin with the profilaggrin gene mutation S2889X or K422X. In the same manner as the results in FIG. 2, each skin was stained with Hematoxylin-Eosin (left side: HE stain), or with the antibody specific to a profilaggrin C-terminal domain and a chromogenic substrate DAB (3,3'-diaminobenzidine) (right side: FLG-C), and the results are shown in FIG. 3. The granular layer was not stained with DAB unlike the results in FIG. 2. Namely, it was revealed that the profilaggrin C-terminal was not formed by the mutation in the atopic dermatitis skin with the mutation.

The aforementioned results have revealed that the antibody specific to the profilaggrin gene C-terminal domain could be used to simply detect the presence or absence of filaggrin gene mutations known to be many, without verifying every mutation by sequencing, and thereby to judge that the skin of a subject is suffering or is highly likely to suffer from atopic dermatitis.

## Claims

1. A method for detecting a mutation in a filaggrin gene, comprising the steps of:
detecting the presence or absence of a C-terminal domain of a human profilaggrin protein in a skin sample of a subject with an antibody specific to said C-terminal domain, wherein the C-terminal domain is a peptide comprising the amino acid sequence set forth in SEQ ID NO:1, and
judging that the mutations in a filaggrin gene is present in the subject, in case when said C-terminal domain is not detected,
wherein said skin sample is a horny layer sample obtained by tape stripping.

2. The method according to claim 1, comprising judging that the subject is suffering or is highly likely to suffer from atopic dermatitis, in case when said C-terminal domain is not detected.

## Patentansprüche

1. Verfahren zur Detektion einer Mutation in einem Filaggrin-Gen, umfassend die Schritte:
Detektieren der Anwesenheit oder Abwesenheit einer C-terminalen Domäne eines humanen Profilaggrin-Proteins in einer Hautprobe eines Subjekts mittels eines für die C-terminale Domäne spezifischen Antikörpers, wobei es sich bei der C-terminalen Domäne um ein Peptid handelt, welches die in SEQ ID NO: 1 dargestellte Aminosäuresequenz umfasst, und
Annehmen, dass die Mutation in einem Filaggrin-Gen in dem Subjekt vorhanden ist, wenn die C-terminale Domäne nicht detektiert wird,
wobei es sich bei der Hautprobe um eine Hornschichtprobe handelt, welche durch Klebefilmabriss erhalten worden ist.

2. Verfahren nach Anspruch 1, umfassend das Annehmen, dass das Subjekt an atopischer Dermatitis leidet oder mit hoher Wahrscheinlichkeit an atopischer Detrmatitis leiden wird, wenn die C-terminale Domäne nicht detektiert wird.

## Revendications

1. Procédé pour détecter une mutation dans un gène de filaggrine, comprenant les étapes de :
détecter la présence ou l'absence d'un domaine C-terminal d'une protéine de profilaggrine humaine dans un échantillon de peau d'un sujet avec un anticorps spécifique dudit domaine C-terminal, **caractérisé en ce que** le domaine C-terminal est un peptide comprenant la séquence d'acide aminé donnée dans SEQ ID NO:1, et
déterminer si les mutations dans un gène de filaggrine sont présentes chez le sujet, dans le cas où ledit domaine C-terminal n'est pas détecté,
**caractérisé en ce que** ledit échantillon de peau est un échantillon de couche cornée obtenu par détachement par bande.

2. Procédé selon la revendication 1, comprenant la détermination qu'un sujet souffre ou a une forte probabilité de souffrir de dermatite atopique, dans le cas où ledit domaine C-terminal n'est pas détecté.


### SEQUENCE LISTING

<110> Shiseido Ltd
<120> Antibody Specific for Profilaggrin C Terminal Domain and Its Use
<130> AA637-PCT
<150> JP2011-245483
   <151> 2011-11-09
<160> 2
<170> PatentIn version 3.1
<210> 1
   <211> 22
   <212> PRT
   <213> Homo sapiens
<400> 1
   Cys Lys Ala Ser Ala Phe Gly Lys Asp His Pro Arg Tyr Tyr Ala Thr
   1               5                   10                  15
   Tyr Ile Asn Lys Asp Pro
               20
<210> 2
   <211> 157
   <212> PRT
   <213> Homo sapiens
<400> 2
   Ser His Arg Asp Thr Ala Ser His Val Gln Ser Ser Pro Val Gln Ser
   1               5                   10                  15
   Asp Ser Ser Thr Ala Lys Glu His Gly His Phe Ser Ser Leu Ser Gln
               20                  25                  30
   Asp Ser Ala Tyr His Ser Gly Ile Gln Ser Arg Gly Ser Pro His Ser
           35                  40                  45
   Ser Ser Ser Tyr His Tyr Gln Ser Glu Gly Thr Glu Arg Gln Lys Gly
       50                  55                  60
   Gln Ser Gly Leu Val Trp Arg His Gly Ser Tyr Gly Ser Ala Asp Tyr
   65                  70                  75                  80
   Asp Tyr Gly Glu Ser Gly Phe Arg His Ser Gln His Gly Ser Val Ser
                   85                  90                  95
   Tyr Asn Ser Asn Pro Val Val Phe Lys Glu Arg Ser Asp Ile Cys Lys
               100                 105                 110
   Ala Ser Ala Phe Gly Lys Asp His Pro Arg Tyr Tyr Ala Thr Tyr Ile
           115                 120                 125
   Asn Lys Asp Pro Gly Leu Cys Gly His Ser Ser Asp Ile Ser Lys Gln
       130                 135                 140
   Leu Gly Phe Ser Gln Ser Gln Arg Tyr Tyr Tyr Tyr Glu
   145                 150                 155